# EUROPEAN PATENT SPECIFICATION

(11) **EP 3 081 079 B1**
(45) Date of publication and mention of the grant of the patent: **22.08.2018**
(21) Application number: 14805015.6
(22) Date of filing: 17.01.2014
(51) Int. Cl.: A01K 23/00, A01K 1/015

(54) **AROMATIC PARTICLES, AND TOILET SAND FOR ANIMALS**
AROMATISCHE PARTIKEL UND STREU FÜR TIERE
PARTICULES AROMATIQUES ET SABLE POUR BOÎTE À LITIÈRE POUR ANIMAUX

(30) Priority: 10.12.2013 JP 2013255050
(43) Date of publication of application: 19.10.2016
(73) Proprietor: Unicharm Corporation, Ehime 799-0111 (JP)
(72) Inventor: TAKAGI, Chiyo, Kanonji-shi Kagawa 769-1602 (JP); KANEKO, Shinya, Kanonji-shi Kagawa 769-1602 (JP)
(74) Representative: Staeger & Sperling Partnerschaftsgesellschaft mbB
(86) International application number: PCT/JP2014/050852
(87) International publication number: WO 2014/192323

(56) References cited:
- EP-A1- 0 410 576
- EP-A1- 2 213 162
- WO-A1-2009/066717
- JP-A- H03 130 024
- JP-A- 2009 153 468
- JP-A- 2013 220 381
- JP-A- 2013 220 381
- JP-U- 3 154 924
- US-A1- 2004 079 293

## Description

### TECHNICAL FIELD

The present invention relates to an aromatic granular material and animal litter.

### BACKGROUND ART

Conventionally, a litter box that includes: a box-shaped litter container with an open top, and animal litter composed of a plurality of granular materials contained in the litter box container, has been used as a toilet for animals kept as pets such as cats and dogs. The granular materials of the animal litter may contain a fragrance for the purpose of masking the odor of feces and urine of pets.

More specifically, animal litter is known, in which a fragrance is sprayed on water absorptive granular materials, such that the granular materials hold the fragrance (for example, see Patent Document 1).

Patent Document 1: Japanese Unexamined Patent Application, Publication No. 2004-33047
Further prior art in this technical field is disclosed in documents US 2004/079293 A1, EP 2 213 162 A1, EP 0 410 576 A1 and JP 2013 220381 A.

### DISCLOSURE OF THE INVENTION

### Problems to be Solved by the Invention

However, in a case of the animal litter in which the fragrance is sprayed and carried onto the water absorptive granular materials, the fragrance together with urine in contact with the surface of the granular materials may soak into the inside of the granular materials, suppressing the generation of an aroma. In addition, urine in contact with the surface of the water absorptive granular materials may soak into the inside of the granular materials, and decay to generate a bad odor.

Therefore, an object of the present invention is to provide an aromatic granular material and animal litter, which are favorable for generating an aroma and are capable of effectively suppressing generation of a bad odor.

### Means for Solving the Problems

The present invention relates to an aromatic granular material according to claim 1 to be used for animal litter including a plurality of granular materials, in which the aromatic granular material includes: a water absorptive core; a waterstop coating layer being formed on a surface of the core, and being composed of a waterstop material which is less water absorptive than the core; and a fragrance layer including a fragrance and being formed on a surface of the waterstop coating layer.

The fragrance layer further includes a dispersing agent capable of dispersing the fragrance into water.

The dispersing agent is a substance being liquid at room temperature, and is composed by including at least one selected from the group consisting of polypropylene glycol, propylene glycol, 1,4-butylene glycol, polyethylene glycol, 1,3-butylene glycol, and dipropylene glycol.

It is preferable that the waterstop material has water absorptivity, and has viscosity in a state of having absorbed water.

The waterstop material is composed by including at least one of polyvinyl alcohol and ethylene-vinyl acetate copolymer.

The waterstop material is composed by including at least one selected from the group consisting of paraffin, polyethylene and polypropylene.

The waterstop coating layer includes inorganic powder.

The present invention also relates to animal litter composed of the aromatic granular material.

The present invention also relates to animal litter, including: a water absorptive granular material including a water absorptive core; and the aromatic granular material, in which content of the aromatic granular material is 0.1 to 30% by volume.

The present invention also relates to animal litter, including: a liquid-permeable fragrance-free granular material; and the aromatic granular material, in which content of the aromatic granular material is 0.1 to 30% by volume.

### Effects of the Invention

The present invention provides an aromatic granular material and animal litter, which are favorable in generating an aroma, and are capable of effectively suppressing generation of a bad odor.

### BRIEF DESCRIPTION OF THE DRAWINGS

FIG. 1 is a perspective view of aromatic granular materials according to one embodiment of the present invention; and
FIG. 2 is a diagram schematically illustrating a cross section of the aromatic granular material according to the above embodiment.

### PREFERRED MODE FOR CARRYING OUT THE INVENTION

### Aromatic Granular Material

A preferred embodiment of aromatic granular materials of the present invention is hereinafter described with reference to the drawings. In the present embodiment, "liquid" including urine is matter excreted from an animal, and includes all fluids which may come in contact with the aromatic granular materials according to the present embodiment.

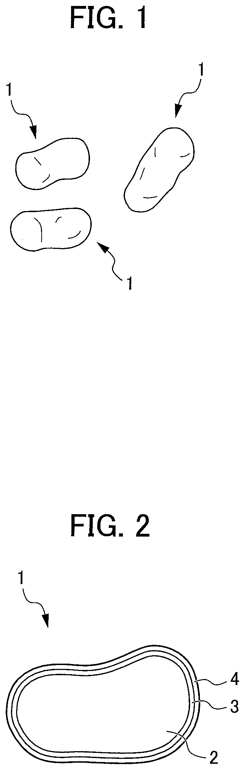

FIG. 1 is a perspective view of aromatic granular materials 1 according to the present embodiment. FIG. 2 is a diagram schematically illustrating a cross section of the aromatic granular material according to the present embodiment.

The aromatic granular material 1 of the present embodiment is a granular material used for animal litter, which includes a plurality of granular materials. As shown in FIG. 1, each of the aromatic granular materials 1 has a distorted spherical shape.

As shown in FIG. 2, the aromatic granular material 1 includes a core 2, a waterstop coating layer 3, and a fragrance layer 4.

The core 2 has water absorptivity. A material composing the core 2 is not limited in particular, as long as water absorptivity is provided; and a plant-derived material such as fiber crushed from paper, pulp, wood flour, or waste paper, an inorganic porous material such as zeolite, silica gel, sepiolite, or attapulgite can be applied thereto. It is preferable that paper or pulp is used for the material composing the core 2, since paper or pulp can suppress the cost in manufacturing the aromatic granular material 1, and is easy to dispose of.

The core 2 may include other materials in addition to materials having water absorptivity. Examples of the other materials may include deodorant.

Examples of the deodorant may include a neutralizing substance such as citric acid or sodium bicarbonate, and an antimicrobial agent. For the antimicrobial agent, organic, inorganic-metallic, photocatalytic, or natural antimicrobial agent can be used; and an organic surface-active antimicrobial agent can be preferably used. Even if the core 2 has absorbed urine, the use of such an antimicrobial agent can suppress proliferation of bacteria. The waterstop coating layer 3 is formed on the surface of the core 2. The waterstop coating layer 3 is composed of a waterstop material which is less water absorptive than the core 2. Here, the water absorptivity is evaluated by an amount of fluid that can be retained per unit mass of a sample.

It is preferable that a waterstop material that is insoluble or hardly soluble in water is used for the waterstop material composing the waterstop coating layer 3. When the waterstop material that is insoluble or hardly soluble in water is used for the waterstop material composing the waterstop coating layer 3, the waterstop coating layer 3 repels water; therefore, a liquid in contact with the aromatic granular material 1 can be prevented from soaking into the core 2. As long as the liquid in contact with the aromatic granular material 1 can be prevented from soaking into the core 2, the liquid does not decay and generate a bad odor inside the core 2. Examples of the material composing the waterstop material that is insoluble in water are polyethylene (PE), polypropylene (PP) and paraffin. When the waterstop material composing the waterstop coating layer 3 is composed by including at least one selected from the group consisting of paraffin, polyethylene and polypropylene, the liquid in contact with the aromatic granular material 1 can be more efficiently prevented from soaking into the core 2.

It is also preferable that a waterstop material having water absorptivity and having viscosity in a state of having absorbed water is used for the waterstop material composing the waterstop coating layer 3. In a case in which the waterstop material having water absorptivity and having viscosity in a state of having absorbed water, is used for the waterstop material composing the waterstop coating layer 3, much of the liquid in contact with the aromatic granular material 1 can be prevented from soaking into the inside of the core 2; and even if a small amount of liquid remains on the surface of the core 2, the liquid is absorbed into the waterstop material, and will be captured into the inside of the core 2 over time. In this manner, by using the waterstop material having water absorptivity and having viscosity in a state of having absorbed water, a bad odor can be prevented from being generated due to decay of the small amount of liquid remaining on the surface of the aromatic granular material 1. Examples of the waterstop material that has water absorptivity and viscosity in a state of having absorbed water are ethylene-vinyl acetate copolymer (EVA), polyvinyl alcohol (PVA). When the waterstop material composing the waterstop coating layer 3 is composed by including at least one of PVA and EVA, much of the liquid in contact with the aromatic granular material 1 can be prevented from soaking into the inside of the core 2; and even if the small amount of liquid remains on the surface of the core 2, the liquid can be efficiently captured into the inside of the core 2.

Here, the waterstop material "having water absorptivity" means that the waterstop material can absorb water of at least 1 mL per unit mass (1 g). It is possible to determine whether the material "has water absorptivity", through the amount of water absorbed into the material, by soaking a sample of 50 g in water of 300 mL to be left unattended at room temperature (25°C), and measuring increase in mass of the sample 10 minutes later.

The waterstop material has viscosity in a state of having absorbed water. Here, "having viscosity in a state of having absorbed water" means having liquidity in a state of having absorbed water. More specifically, in a case in which a waterstop material of 10 g is dissolved or dispersed into water of 1 L, and viscosity of the liquid at room temperature is at least 1.004 mPa · s, the waterstop material is recognized as "having viscosity in a state of having absorbed water".

The waterstop coating layer 3 includes inorganic powder.

Examples of the inorganic powder contained in the waterstop coating layer 3 may include talc, mica, kaolin, calcium carbonate, aluminum oxide. The waterstop coating layer 3 may include other components such as color pigment, as necessary.

The fragrance layer 4 is formed on the surface of the waterstop coating layer. 3. The fragrance layer 4 includes a fragrance.

The fragrance included in the fragrance layer 4 is not limited in particular as long as the fragrance is an aromatic compound; and alcohols such as geraniol, citronellol, citral, eugenol, phenethyl alcohol, thymol, linalool, leaf alcohol, menthol, and benzyl alcohol, and aldehydes such as hexylcinnamaldehyde can be preferably used.

The fragrance layer 4 further includes a dispersing agent capable of dispersing the fragrance into water.

Examples of the component composing the dispersing agent may include glycol and its polymers, which are substances that are liquid at room temperature (25°C). Among these, it is preferable that the dispersing agent is composed by including at least one selected from the group consisting of polypropylene glycol, propylene glycol, 1,4-butylene glycol, polyethylene glycol, 1,3-butylene glycol, and dipropylene glycol. These compounds can disperse various types of fragrances into water, and are therefore versatile. When a polymer of glycol is used as a component composing the dispersing agent, a polymer having molecular weight, which is liquid at room temperature, is selected.

It is preferable that the aromatic granular material 1 in the present embodiment is a spherical or distorted-sphere shaped grain with a diameter of 5 to 30 mm. If the aromatic granular material 1 with such a size and shape is used, cats, etc. would be less reluctant to excrete on the animal litter.

It is preferable that average mass of the aromatic granular material 1 is 50 to 1200 g/L. If the aromatic granular material 1 within such a range of average mass is used, cats, etc. would be less reluctant to excrete on the animal litter.

### Method for Manufacturing Aromatic Granular Material

Next, a method for manufacturing the aromatic granular material 1 according to the present embodiment is described; however, the method for manufacturing the aromatic granular material 1 is not limited thereto, and can be modified as appropriate.

The aromatic granular material 1 is manufactured through a granulating step S1, a waterstop coating step S2, a drying step S3, and a fragrance coating step S4.

In the granulating step S1, the core 2 is granulated, by developing and rolling the material composing the core 2 on a rotary table or inside a drum. In the granulating step S1, it is preferable that the material composing the core 2 is formed without being compressed.

In the waterstop coating step S2, a waterstop material is coated on the surface of the core 2.

The waterstop material is dissolved or dispersed into water or organic solvent in advance. A solution or dispersion of the waterstop material is sprayed with a spray onto the surface of the core 2 to cause the waterstop material to adhere to the surface of the core 2. The inorganic powder is dispersed into the solution or dispersion of the waterstop material, as necessary.

In the drying step S3, the core 2 is dried, onto the surface of which the solution or dispersion of the waterstop material has adhered. In the drying step S3, it is preferable that the core 2 is dried by using a dryer. In the drying step S3, the water or organic solvent evaporates, and the waterstop coating layer 3 is formed on the surface of the core 2.

In the fragrance coating step S4, the fragrance layer 4 is formed on the surface of the waterstop coating layer 3. In the fragrance coating step S4, the fragrance is sprayed with a spray, so as to be brought in contact with the surface of the waterstop coating layer 3.

In the fragrance coating step S4, it is preferable that a solution or dispersion of the fragrance is prepared in advance by blending the fragrance with the dispersing agent. As a result, the fragrance component can be stably dispersed into the dispersing agent; therefore, handling of the fragrance dispersion is improved, and in addition, it is possible to enhance the generation of an aroma when a liquid comes in contact with the fragrance layer 4, as will be described later in detail. At this time, by using the aforementioned substances (glycol and its polymers), which are liquid at room temperature, as the dispersing agent, it is possible to prevent the fragrance from evaporating when blending the fragrance with the dispersing agent. Furthermore, by blending the aforementioned substances (glycol and its polymers), which are liquid at room temperature, as the dispersing agent into the fragrance, the mixture of the fragrance and the dispersing agent does not need to be heated and melted in the fragrance coating step S4, and it is possible to prevent the fragrance from evaporating due to heating.

### Aspects of Using Aromatic Granular Material

Next, first to third aspects of using the aromatic granular material according to the present embodiment are described.

In the first aspect of using the aromatic granular material 1, the aromatic granular materials 1 are laid in an animal litter box.

In the first aspect, the aromatic granular materials 1 are laid in an animal litter box, thereby making it possible to suppress a bad odor generated from liquids such as animal urine.

In the second aspect of using the aromatic granular material 1, the aromatic granular materials 1 are blended with water absorptive granular materials including water absorptive cores, and are laid in an animal litter box. As will be described later in detail, the water absorptive granular materials absorb liquid to form agglomerate with adjacent water absorptive granular materials.

In the second aspect of use, the aromatic granular materials 1 are blended with the water absorptive granular materials that absorb liquid to form agglomerate, and are laid in an animal litter box, thereby making it possible to suppress a bad odor generated from liquids such as animal urine.

In the third aspect of using the aromatic granular material 1, the aromatic granular materials 1 are blended with liquid-permeable fragrance-free granular materials, and are laid in an animal litter box. As will be described later in detail, the fragrance-free granular materials allow much of the liquids in contact therewith to quickly flow downward.

In the third aspect of use, the aromatic granular materials 1 are blended with the liquid-permeable granular materials and are laid in an animal litter box, thereby making it possible to suppress a bad odor generated from liquids such as animal urine.

In a case in which the aromatic granular materials 1 are used as blended with other granular materials, as in the second and third aspects of use, the amount of blending the aromatic granular materials 1 can be adjusted as appropriate, in consideration of the generation of an aroma from an animal litter box.

### Animal Litter

Next, a description is given of first to third animal litter using the aromatic granular materials 1 according to the present embodiment. Animals using such animal litter include so-called pets such as dogs, cats, rabbits, and hamsters, as well as cubs of animals such as tigers and lions.

The first animal litter is composed of aromatic granular materials 1. In other words, the first animal litter is the animal litter composed of the aromatic granular materials 1 to be laid in the animal litter box in the first aspect of use. It is preferable that the first animal litter is composed of only the aromatic granular materials 1.

The second animal litter includes: water absorptive granular materials including water absorptive cores; and the aromatic granular materials 1. In other words, the second animal litter is animal litter to be laid in the animal litter box in the second aspect of use, and is animal litter in which the water absorptive granular materials including water absorptive cores are blended beforehand with the aromatic granular materials 1.

The water absorptive cores of the water absorptive granular materials may be composed of inorganic porous materials such as bentonite, or may be composed of organic fiber materials such as pulp. The cores may be composed of organic fiber materials such as pulp; and a covering layer composed by including superabsorbent polymers may be formed on the surface of the cores. Here, examples of the superabsorbent polymers may include polyacrylic-acid-based polymers, starch-acrylic-acid-based polymers, etc.

The water absorptive granular materials absorb liquid to form agglomerate with adjacent water absorptive granular materials. Therefore, the second animal litter can be preferably used as a type of litter in which a plurality of water absorptive granular materials absorb liquid to form agglomerate, which can be removed thereafter.

In a case in which the second animal litter is manufactured, it is preferable that content of the aromatic granular materials 1 is 0.1 to 30% by volume in the second animal litter. If the content of the aromatic granular materials 1 is less than 0.1% by volume in the second animal litter, the generation of an aroma from the second animal litter is suppressed; and if the content is more than 30% by volume, the water absorptive granular materials are relatively small in amount, making it difficult to form agglomerate by the water absorptive granular materials that have absorbed liquid. The content of the aromatic granular materials 1 in the animal litter is defined on a volumetric basis. As used herein, "% by volume" is a percentage represented by proportion of capacity (volume) including gaps between the granular materials when filled with only particular granular materials (the aromatic granular materials 1), with respect to the entire capacity (volume) including gaps between the granular materials when filled with all the granular materials of the animal litter.

The third animal litter includes the liquid-permeable fragrance-free granular materials and the aromatic granular materials 1. In other words, the third animal litter is animal litter to be laid in the animal litter box in the third aspect of use, and is animal litter in which the liquid-permeable fragrance-free granular materials are blended beforehand with the aromatic granular materials 1.

Examples of the liquid-permeable fragrance-free granular materials may include granular materials hardened from silica gel or zeolite, or wooden granular materials containing resin. For the liquid-permeable fragrance-free granular materials, it is also possible to use a granular material including: a water absorptive core, and a waterstop coating layer formed on the surface of the core and composed of a waterstop material which is less water absorptive than the core. The material composing the core of the fragrance-free granular materials may be similar to the material composing the core 2 of the aromatic granular material 1. The material composing the waterstop coating layer of the fragrance-free granular material may be similar to the material composing the waterstop coating layer 3 of the aromatic granular material 1.

The third animal litter is liquid-permeable, and therefore allows much of the liquids in contact therewith to quickly flow downward. Therefore, the third animal litter can be preferably used as a type of animal litter that is laid in an upper container of a so-called integrated animal litter box system, which includes: the upper container with a plurality of holes provided in a bottom face, and a lower container which is arranged below the upper container and accommodates a liquid-absorbing member.

In a case in which the third animal litter is manufactured, it is preferable that content of the aromatic granular materials 1 is 0.1 to 30% by volume in the third litter. If the content of the aromatic granular materials 1 is less than 0.1% by volume in the third animal litter, the generation of an aroma from the second animal litter is suppressed; and if the content is more than 30% by volume, the consumption of the fragrance is increased to raise the cost of manufacturing the third animal litter.

The following effects are achieved by the aromatic granular material 1, and the animal litter using the aromatic granular material 1, according to the present embodiment described above.

In the aromatic granular material 1 of the present embodiment, the waterstop coating layer 3 composed of the waterstop material is formed on the surface of the water absorptive core 2; and the fragrance layer 4 including the fragrance is further formed on the surface of the waterstop coating layer 3.

As a result, the liquid in contact with the surface of the aromatic granular material 1 is unlikely to be captured inwardly beyond the waterstop coating layer 3, and much of the liquid remains on the fragrance layer 4. The liquid remaining on the fragrance layer 4 wets the surface of the aromatic granular material 1. The wetted portion on the surface of the aromatic granular material 1 facilitates emission of aroma of the fragrance of the fragrance layer 4, thereby improving the generation of an aroma from the aromatic granular material 1.

The presence of the waterstop coating layer 3 can suppress a bad odor that would be generated if much of the liquid in contact with the surface of the aromatic granular material 1 is captured to decay inside the core 2. Furthermore, the formation of the fragrance layer 4 on the surface of the waterstop coating layer 3 can suppress deterioration of the aroma generation from the aromatic granular material 1, which would be caused if the fragrance is captured inside the core 2 by the liquid in contact with the surface of the aromatic granular material 1.

In the present embodiment, the fragrance layer 4 contains a substance that is liquid at room temperature (25°C), and is a dispersing agent capable of dispersing a fragrance into water.

As a result, when a liquid comes in contact with the surface of the aromatic granular material 1, the dispersing agent disperses the fragrance of the fragrance layer 4 into the liquid, thereby facilitating the emission of an aroma of the fragrance from the fragrance layer 4. Therefore, the generation of an aroma from the aromatic granular material 1 is further improved.

Furthermore, since the dispersing agent has an affinity for liquids, the liquid in contact with the surface of the aromatic granular material 1 wets a large area on the surface of the aromatic granular material 1. When the large area on the surface of the aromatic granular material 1 gets wet, the aroma of the fragrance is easily emitted from the larger area, thereby further improving the generation of an aroma from the aromatic granular material 1.

In the present embodiment, the dispersing agent contained in the fragrance layer 4 is composed by including at least one selected from the group consisting of polypropylene glycol, propylene glycol, 1,4-butylene glycol, polyethylene glycol, 1,3-butylene glycol, and dipropylene glycol.

As described above, these compounds can disperse various types of fragrances into water, and are therefore versatile.

In the present embodiment, the waterstop material composing the waterstop coating layer 3 has water absorptivity and has viscosity in a state of having absorbed water.

As a result, much of the liquid in contact with the aromatic granular material 1 can be prevented from soaking into the inside of the core 2; and even if a small amount of liquid remains on the surface of the core 2, the liquid is absorbed into the waterstop material, and will be captured to the inside of the core 2 over time. Therefore, a bad odor can be prevented from being generated due to decay of the small amount of liquid remaining on the surface of the aromatic granular material 1.

In a type of animal litter that is laid in an upper container of an integrated animal litter box system, it is strongly desired to prevent a bad odor from generating due to decay of the small amount of liquid remaining on the surface of the granular materials. Therefore, the aromatic granular material 1, which includes the waterstop coating layer 3 composed by using the waterstop material that has water absorptivity and has viscosity in a state of having absorbed water, can be preferably used for the type of animal litter that is laid in an upper container of an integrated animal litter box system.

In the present embodiment, the waterstop material composing the waterstop coating layer 3 is composed by including at least one of polyvinyl alcohol and ethylene-vinyl acetate copolymer.

As a result, generation of a bad odor from the aromatic granular material 1 can be further suppressed.

In the present embodiment, the waterstop material composing the waterstop coating layer 3 is composed by including at least one selected from the group consisting of paraffin, polyethylene and polypropylene.

As a result, the generation of an aroma from the aromatic granular material 1 is further improved, and the generation of a bad odor can also be suppressed.

In the present embodiment, the waterstop coating layer 3 contains inorganic powder.

As a result, the inorganic powder gets into irregularities on the surface of the water absorptive core 2, so that interstices on the surface of the core 2 are filled. Since the interstices on the surface of the core 2 are filled, the liquid is unlikely to be absorbed into the core 2, and the waterstop property of the waterstop coating layer 3 formed on the surface of the core 2 is improved.

Since the inorganic powder gets into the irregularities on the surface of the water absorptive core 2, the state of the surface of the core 2 can be smoothed. If the state of the surface of the core 2 can be smoothed, the waterstop coating layer 3 can be uniformly and evenly formed on the surface of the core 2. If the thickness of the waterstop coating layer 3 is uneven, liquid would easily soak thereinto from a thin portion of the waterstop coating layer 3; however, since the waterstop coating layer 3 is uniformly and evenly formed on the surface of the core 2, the soaking of the liquid into the inside of the core 2 can be further suppressed.

If the waterstop coating layer 3 can be uniformly and evenly formed on the surface of the core 2, it is possible to reduce the uneven coloring when the waterstop coating layer 3 contains color pigment.

The first animal litter is composed of the aromatic granular materials 1.

As a result, it is possible to provide animal litter that is favorable for the generation of an aroma, and is capable of suppressing generation of a bad odor.

The second animal litter includes the water absorptive granular materials and the aromatic granular materials 1, in which the content of the aromatic granular materials is 0.1 to 30% by volume. As described above, in the second animal litter, the superabsorbent polymer absorbs liquid to form agglomerate with adjacent water absorptive granular materials. In this manner, the second animal litter can be preferably used as a type of animal litter, in which a plurality of water absorptive granular materials absorb liquid to form agglomerate, which can be removed thereafter.

Water absorptive granular materials can easily capture fragrances inside; however, since the aromatic granular materials 1 are blended with the water absorptive granular materials in the second animal litter, the generation of an aroma can be enhanced in a type of animal litter, in which a plurality of water absorptive granular materials absorb liquid to form agglomerate.

In the second animal litter, since the aromatic granular materials 1 are in contact with the water absorptive granular materials, the fragrance of the fragrance layer 4 is also attached to the surface of the water absorptive granular materials. By such an action, the fragrance is spread throughout the second animal litter.

Therefore, the second animal litter can enhance the generation of an aroma, while curtailing the amount of fragrance used.

The third animal litter includes the liquid-permeable fragrance-free granular materials and the aromatic granular materials 1, in which the content of the aromatic granular materials is 0.1 to 30% by volume. As described above, the third animal litter can be preferably used for a type of animal litter that is laid in an upper container of an integrated animal litter box system.

In this manner, since the aromatic granular materials 1 are blended with non-water-absorptive fragrance-free granular materials, the generation of an aroma can be enhanced in the animal litter for the integrated animal litter box system.

In the third animal litter, since the aromatic granular materials 1 are in contact with the fragrance-free granular materials, the fragrance of the fragrance layer 4 is also attached to the surface of the fragrance-free absorptive granular materials. By such an action, the fragrance is spread throughout the third animal litter. Therefore, the third animal litter can enhance the generation of an aroma, while curtailing the amount of fragrance used.

The aromatic granular material 1 and the animal litter using the aromatic granular material 1 according to one embodiment of the present invention have been described above.

### EXPLANATION OF REFERENCE NUMERALS

1 aromatic granular material
2 core
3 waterstop coating layer
4 fragrance layer

## Claims

1. An aromatic granular material (1) to be used in animal litter including a plurality of granular materials, the aromatic granular material comprising:
a water absorptive core (2);
a waterstop coating layer (3) being formed on a surface of the core, and being composed of a waterstop material which is less water absorptive than the core; and
a fragrance layer (4) including a fragrance and being formed on a surface of the waterstop coating layer, wherein the fragrance layer further includes a dispersing agent capable of dispersing the fragrance into water, the dispersing agent is a substance being liquid at room temperature, and is composed by including at least one selected from the group consisting of polypropylene glycol, propylene glycol, 1,4-butylene glycol, polyethylene glycol, 1,3-butylene glycol, and dipropylene glycol,
**characterized in that**
the waterstop material of the waterstop coating layer (3) is composed by including at least one selected from the group consisting of paraffin, polyethylene and polypropylene or is composed by including at least one of polyvinyl alcohol and ethylene-vinyl acetate copolymer, and wherein the waterstop coating layer includes inorganic powder capable of getting into irregularities on the surface of the water absorptive core.

2. The aromatic granular material according to claim 1, wherein the inorganic powder is composed by including at least one selected from talc, mica, kaolin, calcium carbonate and aluminum oxide.

3. Animal litter comprising the aromatic granular material (1) according to claim 1 or 2.

4. Animal litter, comprising:
a water absorptive granular material including a water absorptive core; and
the aromatic granular material (1) according to claim 1 or 2,
wherein content of the aromatic granular material is 0,1 to 30% by volume.

5. Animal litter, comprising:
a liquid-permeable fragrance-free granular material; and
the aromatic granular material (1) according to claims 1 or 2, wherein content of the aromatic granular material is 0,1 to 30% by volume.

## Patentansprüche

1. Ein aromatisches Granulat (1) zur Verwendung in Tierstreu, das eine Vielzahl von Granulaten umfasst, das aromatische Granulat umfassend:
einen wasserabsorbierenden Kern (2);
eine Wasserstopbeschichtungsschicht (3), die auf einer Oberfläche des Kerns ausgebildet ist, und aus einem Wasserstopmaterial, das weniger wasserabsorbierend ist als der Kern, gebildet ist; und
eine Duftstoffschicht (4), die einen Duftstoff umfasst und auf einer Oberfläche der Wasserstopbeschichtungsschicht ausgebildet ist, wobei die Duftstoffschicht ferner ein Dispersionsmedium, das geeignet ist den Duftstoff in Wasser zu lösen, umfasst, das Dispersionsmedium eine Substanz Ist, die bei Raumtemperatur flüssig ist, und durch Aufnahme wenigstens einer, die aus der Gruppe bestehend aus Polypropylenglykol, Propylenglykol, 1,4-Butyleneglykol, Polyethleneglykol, 1,3-Butylenglykol, und Dipropyleneglykol ausgewählt wird, ausgebildet ist,
**dadurch gekennzeichnet, dass**
das Wasserstopmaterial der Wasserstopbeschichtungsschicht (3) durch Aufnahme wenigstens einem, das aus der Gruppe bestehend aus Paraffinm, Polyethylene und Polypropylen ausgewählt wird, ausgebildet ist oder durch Aufnahme wenigstens einem von Polyvinylalkohol und Ethylen-Vinylacetat Copolymer ausgebildet ist, und wobei die Wasserstopbeschichtungsschicht anorganisches Pulver, das geeignet ist sich in Unregelmäßigkeiten auf der Oberfläche des wasserabsorbierenden Kerns einzulassen, umfasst.

2. Das aromatische Granulat gemäß Anspruch 1, wobei das anorganische Pulver durch Aufnahme wenigstens eines, das aus Talk, Glimmer, Kaolin, Kalziumkarbonat und Aluminiumoxid ausgewählt wird, ausgebildet ist.

3. Tierstreu, das das aromatische Granulat (1) gemäß Anspruch 1 oder 2 umfasst.

4. Tierstreu, umfassend:
ein wasserabsorbierendes Granulat, das einen wasserabsorbierenden Kern umfasst; und
das aromatische Granulat (1) gemäß Anspruch 1 oder 2,
wobei Gehalt des aromatischen Granulats 0,1 bis 30 Volumenprozent beträgt.

5. Tierstreu, umfassend:
ein flüssigkeitsdurchlässiges duftstofffreies Granulat; und
das aromatische Granulat (1) gemäß Anspruch 1 oder 2, wobei Gehalt des aromatischen Granulats 0,1 bis 30 Volumenprozent beträgt.

## Revendications

1. Matière granulaire aromatique (1) destinée à être utilisée dans une litière pour animaux incluant une pluralité de matières granulaires, la matière granulaire aromatique comprenant :
un noyau d'absorption d'eau (2) ;
une couche de revêtement étanche à l'eau (3) formée sur une surface du noyau, et étant composée d'une matière étanche à l'eau qui a un pouvoir d'absorption d'eau inférieur à celui du noyau ; et
une couche de parfum (4) incluant un parfum et étant formée sur une surface de la couche de revêtement étanche à l'eau, dans laquelle la couche de parfum comporte en outre un agent de dispersion capable de disperser le parfum dans l'eau, l'agent de dispersion étant une substance liquide à température ambiante et composé en incluant au moins l'un choisi dans le groupe constitué de polypropylène glycol, propylène glycol, 1,4-butylène glycol, polyéthylène glycol, 1,3-butylène glycol et dipropylène glycol, **caractérisée en ce que**
la matière étanche à l'eau de la couche de revêtement étanche à l'eau (3) est composée en incluant au moins l'un choisi dans le groupe constitué de paraffine, polyéthylène et polypropylène ou est composée en incluant au moins l'un d'un alcool polyvinylique et d'un copolymère d'éthylène-acétate de vinyle, et dans laquelle la couche de revêtement étanche à l'eau inclut une poudre inorganique capable d'entrer dans des irrégularités sur la surface du noyau d'absorption d'eau.

2. Matière granulaire aromatique selon la revendication 1, dans laquelle la poudre inorganique est composée en incluant au moins l'un choisi parmi le talc, le mica, le kaolin, le carbonate de calcium et l'oxyde d'aluminium.

3. Litière pour animaux comprenant la matière granulaire aromatique (1) selon la revendication 1 ou 2.

4. Litière pour animaux, comprenant :
une matière granulaire d'absorption d'eau incluant un noyau d'absorption d'eau ; et
la matière granulaire aromatique (1) selon la revendication 1 ou 2,
dans laquelle la teneur en matière granulaire aromatique est de 0,1 à 30 % en volume.

5. Litière pour animaux, comprenant :
une matière granulaire sans parfum perméable au liquide ; et
la matière granulaire aromatique (1) selon les revendications 1 ou 2, dans laquelle la teneur en matière granulaire aromatique est de 0,1 à 30 % en volume.
